# EUROPEAN PATENT APPLICATION

(11) **EP 2 380 634 A2**
(43) Date of publication of application: **26.10.2011**
(21) Application number: 09835178.6
(22) Date of filing: 24.11.2009
(51) Int. Cl.: A62B 18/02

(54) **DEVICE FOR PREVENTING MOUTH FROM OPENING DURING SLEEP**

(30) Priority: 26.12.2008 KR 20080134247
(71) Applicant: Ha, Che Hub, Seoul 156-836 (KR)
(72) Inventor: Ha, Che Hub, Seoul 156-836 (KR)
(74) Representative: Rapisardi, Mariacristina
(86) International application number: PCT/KR2009/006944
(87) International publication number: WO 2010/074412

(57) **Abstract**

Provided is a device for preventing mouth opening during sleep including: an upper lip supporter configured to be firmly disposed above an upper lip; a lower lip supporter configured to be firmly disposed below a lower lip; a lower jaw supporter configured to be firmly disposed on a lower surface of a lower jaw and having a protruded fixing unit in a center part, the protruded fixing unit being protruded toward a front side of the lower jaw and bent upward forming a gentle curve along the lower jaw so that the lower jaw supporter does not move toward a neck, and a coupling medium coupling the supporters through coupling holes disposed on both sides of each supporter to maintain a shape of the device, the coupling medium hung on ears when the device is worn so that the device is firmly disposed on a face. According to the device of the present invention, the lower jaw supporter prevents the lower jaw from opening during sleep due to a relaxation of oral muscles, and each of the upper lip supporter and lower lip supporter is disposed above the upper lip and below the lower lip to fix both lips to each other and prevent a mouth from opening. Therefore, when the device is worn during sleep, mouth breathing is inhibited, nose breathing is induced, such sleep disorders as xerostama, stertorous respiration, and sleep apnea are alleviated, and oral muscles are strengthened. In addition, the device minimizes discomfort by minimizing an area of the facial skin which comes into contact with the device when the device is worn. In addition, the device has a simplified structure, is manufactured inexpensively, and may be used as an auxiliary apparatus for alleviating xerostama, stertorous respiration, and sleep apnea.

## Description

### BACKGROUND OF THE INVENTION

The present invention disclosed herein relates to a device for preventing mouth opening during sleep, and more particularly, to a device including: an upper lip supporter configured to be firmly disposed above an upper lip; a lower lip supporter configured to be firmly disposed below a lower lip; a lower jaw supporter configured to be firmly disposed on a lower surface of a lower jaw and having a protruded fixing unit in a center part, the protruded fixing unit being protruded toward a front side of the lower jaw and bent upward forming a gentle curve along the lower jaw so that the lower jaw supporter does not move toward a neck; and a coupling medium coupling the supporters through coupling holes disposed on both sides of each supporter to maintain a shape of the device, the coupling medium hung on cars when the device is worn so that the device is firmly disposed on a face.

In general, snoring refers to an breathing during sleep and is more frequent among people in their thirties or older. It is increasingly regarded as a disease with the development of respiratory physiology. As a symptom of a breathing disorder, it is a sound made when a person breathes abnormally during sleep. Then, the atony of oral muscles results in the root of the tongue blocking part of the respiratory tract, narrowing the tract to increase the speed at which air passes, and vibrating the uvula.

When a person breathes, air passes through such flexible tissues as the throat, uvula, and tonsil. By day, because the human body remains upright, she flexible tissues are placed in right positions to keep the tract wide enough. However, when the person, steeps on the back with the mouth open, the root of the tongue moves back, blocks and narrows the tract. When air passes through the narrowed tract, the speed of the passage increases and the flexible tissues nearby are vibrated to make a snoring sound.

If the respiratory tract is completely blocked due to obesity, a severe atony of the muscles during sleep and so forth, the air temporarily stops flowing into the lungs. Such a symptom is called sleep apnea.

If the condition continues periodically, the lungs cannot bring in atmospheric oxygen and the blood oxygen concentration drops to cause oxygen deficiency. In this case, the possibility of death due to a sleep attack or a heart attack approximately triples.

Snoring during sleep may have various causes, the biggest of which is mouth breathing caused, by an inability to breathe through the nose. According to a research, about 8.5% of snoring patients breathe through the mouth during sleep with their mouths open.

A person who breathes through the mouth not only inhales more air than a person who breathes through the nose, but also breathes cold, dry and dusty air into the lungs without any filtering, heightening the possibility of bacterial infection or microbism. Also, the discharge of carbon dioxide is quickened during an exhalation, and the brain, detecting the acceleration, stimulates goblet cells to make phlegm, slow down respiration and constrict blood vessels. Then, the blood oxygen concentration drops as the time for the lungs to extract oxygen from the inhaled air becomes insufficient. Furthermore, mouth breathing leads to such physical abnormalities as xerostama, somnipathy, headache, chronic fatigue and tooth deformation. Therefore, a person who breathes through the mouth needs to be induced to breathe through the nose.

At present, there are many products to alleviate or eliminate snoring, including anti-snoring pillows and pajamas with a back part filled with air to prevent a person from lying on the back while asleep. However, those products disturb sleep while somewhat, effective against snoring.

Also available are breathing apparatuses to treat sleep apnea, e.g., CPAP and BIPAP. The apparatuses inject air having a predetermined positive pressure correspondingly to breathing by using an oxygen mask in form of a gas mask, and act as an air splint to prevent me respiratory tract from being blocked by negative pressure when air is inhaled.

However, the apparatuses are highly expensive and the ask needs to be worn on an entire face, causing a sense of anxiety, a dried nasal cavity and a nasal obstruction while difficult to be carried. In the meantime, carried out to prevent mouth breathing and induce nose breathing are operations and drug treatments, mouth taping, head strapping to fix the jaw, oral implant insertion, etc. However, those methods cause dermal irritation or insomnia by stimulating the head. Also, those are difficult to be used for a long period of time due to a side effect such as drug addiction while difficult to be popularized due to their economic burden and wearing discomfort.

An anti-snoring mask to induce a forced breathing by means of an air tank placed on the back of a wearer has been suggested. However, since the wearer cannot lie on the back because of the air tank and has to lie on his or her side, organs including the heart receive pressure and the mask inhibits those who usually sleep on the back from having a deep sleep and moving the body freely during sleep.

To resolve the aforementioned, the applicant has suggested a mask for preventing mouth opening during sleep via Registered Utility Model of Korea no. 0405903. However, the mask has a stifling feeling and discomfort when worn, is difficult to wear in summer due to perspiration, and requires a high manufacturing cost. Thus, it has not been used conveniently.

### SUMMARY OF THE INVENTION

The present invention provides a device inhibiting mouth breathing, inducing nose breathing, alleviating such sleep disorders as xerostama, stertorous respiration, and sleep apnea, and strengthening oral muscles without a stifling feeling or a sense of repulsion when worn during sleep.

The present invention also provides a device which minimizes wearing discomfort by minimizing an area of the facial skin coming into contact with the device when the device is worn, and is manufactured inexpensively while efficiently preventing a mouth from opening during sleep.

The present invention also provides a device fitting a face of a wearer and easily worn and separated. Supporters of the device are coupled into one by means of a coupling medium, and distances between the supporters easily adjusted.

According to an exemplary embodiment of the present invention, there is provided a device for preventing mouth opening during sleep, the device including: an upper lip supporter having at least one coupling hole in each of upper and lower side portions, the upper lip supporter configured to be firmly disposed above an upper lip and has a thin plate shape; a lower lip supporter having at least one coupling hole in each of upper and lower side portions, the lower lip supporter configured to be firmly disposed below a lower lip and has a thin plate shape; a lower jaw supporter having a thin plate shape and configured to be firmly disposed on a lower surface of a lower jaw, the lower jaw supporter including a coupling hole in each upper side portion, at least one length adjustment hole in each lower side portion in a length direction of the lower side portion, and a protruded fixing unit in a center part, the protruded fixing unit being protruded toward a front side of the lower jaw and bent upward forming a gentle curve along the lower jaw so that the lower jaw supporter does not move toward a neck, and a coupling medium, a center part of the coupling medium disposed on a rear surface of the lower jaw supporter to support the lower jaw supporter, and each side end of the coupling medium inserted into the coupling hole of the lower jaw supporter toward a front side of the lower jaw supporter and then sequentially inserted into the coupling holes of the lower lip supporter and the upper lip supporter to connect the lower lip supporter and the upper lip supporter upward, and the each side end of the coupling medium inserted into the length adjustment hole disposed in each lower portion of the lower jaw supporter so that a length of the coupling medium hung on each ear is adjusted and the coupling medium is disposed firmly on a face.

Therefore, a long strap may form the coupling medium in entirety. The center part of the coupling medium is fixed to the rear surface of the lower jaw supporter. Then, both side ends of the coupling medium are inserted into coupling holes disposed in both upper side portions of the lower jaw supporter, and passed from the rear surface to a front surface of the lower jaw supporter to supporter the lower jaw supporter. Then, the both side ends are sequentially and alternately inserted into the coupling holes of the lower lip supporter and the upper lip supporter and coupled to couple the supporters. Then, the both side ends are left with an enough length, and are sequentially and alternately inserted into length adjustment holes disposed at both lower side ends of the lower jaw supporter to couple the supporters with the coupling medium. The wearer may adjust the length of the coupling medium by pulling or untying the side ends inserted into the length adjustment holes so that the device is worn and disposed firmly on the facial skin.

In the meantime, each coupling hole disposed on each supporter may have a length larger than a width, and the length, adjustment hole disposed on the lower jaw supporter may have a width larger than a length so that the coupling medium is inserted smoothly into each coupling hole by having a shape corresponding to a direction in which the coupling medium is inserted. In addition, the wearer may pull or untie the coupling medium smoothly to adjust the length of the coupling medium since the length adjustment hole has a shape corresponding to the direction in which the coupling medium is inserted.

In addition, a stopper may be disposed at the each side end of the coupling medium so that the each side end of the coupling medium inserted into the length adjustment hole is not separated from the length adjustment hole. A ring or tweezers having a spring may be used as the stopper. When a strap formed of elastic span fabric is used as the coupling medium, knots at the both side ends of the strap may act as stoppers.

In addition, a guideline may be disposed on the rear surface of the lower jaw supporter to receive the center part of the coupling medium, and the guideline may include a groove having a predetermined depth and a semicircular are shape from the coupling hole toward the neck.

Therefore, when the device is worn on the face, the lower jaw supporter may be firmly fixed on the lower jaw. The center part of the coupling medium may have a circular arc shape starting from both side ends of the lower surface of the lower jaw and convex toward the neck, so that the center part may be fixed to the lower jaw by pressing an inner surface of the lower jaw supporter. Then, the lower jaw supporter may be firmly fixed to the lower jaw since the coupling medium is not drooped toward an end of the jaw or the neck, and the lower jaw supporter may not be easily overturned causing discomfort when the device is worn. In addition, the guideline which has a groove receiving the coupling medium may be disposed on the rear surface of the lower jaw supporter.

In addition, a plurality of holes are disposed across the lower jaw supporter to allow flow of air and improve an appearance of the device.

In the meantime, the each supporter is formed of an elastic and soft synthetic resin material, and the coupling medium is a strap formed of a flexible fabric and having a predetermined thickness. In this case, the elastic and soft synthetic resin material may include polyethylene, a polypropylene resin, and a latex resin. The coupling medium may be a span strap formed of an elastic rubber fiber woven with cotton or a synthetic fiber.

By using such a cotton material, the supporters and the coupling medium of the device may not cause discomfort on the face when the device is worn.

In addition, the each supporter has a forwardly-curved center part corresponding to a shape of the face so as to be firmly disposed on the face.

According to an exemplary embodiment of the present invention, a wearing sub-supporter formed of a natural cotton fabric or a soft velvet woven fabric is disposed on a rear surface of each supporter to reduce an irritation from a dermal contact and to increase wearing comfort. The wearing sub-supporter absorbs moisture and sweat on the face, increases wearing comfort, and firmly disposes the device on the face.

The device according to an embodiment of the present invention further includes a mark preventing unit having a polygonal or oval thin-plate shape. An inner surface of the mark preventing unit comes into contact with a skin of a cheekbone, and a center part of the mark preventing unit is convexly protruded correspondingly to a protruded shape of the cheekbone. A pair of coupling medium fixing protrusions are disposed on both sides of an upper portion of the mark preventing unit, or a single coupling medium fixing protrusion is disposed on a side of the upper portion of the mark preventing unit, for fitting the coupling medium therein. Therefore, when the device is worn on the face during sleep by using the coupling medium, a mark of the coupling medium may not be left on the facial skin. Therefore, after the device is worn and firmly disposed on the face by adjusting the coupling medium, the wearer may dispose the mark preventing unit on the cheekbone, and then the coupling medium may be inserted into the coupling medium fixing protrusions disposed on both sides of an outer surface of the mark preventing unit. In this case, a mark preventing sub-supporter formed of the same material as a material of the wearing sub-supporter may be disposed on the inner surface of the mark preventing unit for wearing comfort. An edge of the mark preventing sub-supporter may be rounded so that the edge has a gentle curve shape, and the edge may be formed of the elastic and soft synthetic resin material.

In case the pair of coupling medium fixing protrusions is used, the coupling medium fixing protrusions are outwardly inclined so that a distance between the pair of coupling medium fixing protrusions increases upward. Therefore, the coupling media having a variety of thicknesses may be firmly inserted into the opening between the coupling medium fixing protrusions. The coupling medium fixing protrusions may be outwardly inclined so that the distance between the pair of coupling medium fixing protrusions increases upward and allows the coupling media having the variety of thicknesses to be used.

In case the single coupling medium fixing protrusions is used, a mark preventing protection cover is disposed in an outer portion for comfortable wearing.

The device according to an embodiment of the present invention, if necessary, includes the mark preventing sub-supporter formed of the natural cotton fabric or the soft velvet woven fabric and disposed on the bottom surface of the mark preventing units.

### BRIEF DESCRIPTION OF THE DRAWINGS

The accompanying drawings are included to provide a further understanding of the present invention, and are incorporated is and constitute a part of this specification. The drawings illustrate exemplary embodiments of the present invention and, together with the description, serve to explain principles of the present invention. In the drawings:

FIGS. 1 through 3 are front views illustrating a device for preventing mouth opening during sleep according to embodiments of the present invention;

FIGS. 4 through 6 are rear views illustrating the device for preventing mouth opening during sleep according to embodiments of the present invention;

FIG. 7 is a side sectional view taken along line A-A' of FIG. 1 to illustrate the device for preventing mouth opening during sleep according to an embodiment of the present invention;

FIG. 8 is a cross sectional view illustrating a mark preventing unit of the device according to an embodiment of the present invention, and FIG. 9 is a vertical sectional view taken along line B-B' of FIG. 9; and

FIGS. 10 through 12 are views illustrating states of the device worn on the face according to the embodiments of the present invention.

### DETAILED DESCRIPTION OF PREFERRED EMBODIMENTS

Hereinafter, it will be described about an exemplary embodiment of a device for preventing mouth opening during sleep in conjunction with the accompanying drawings.

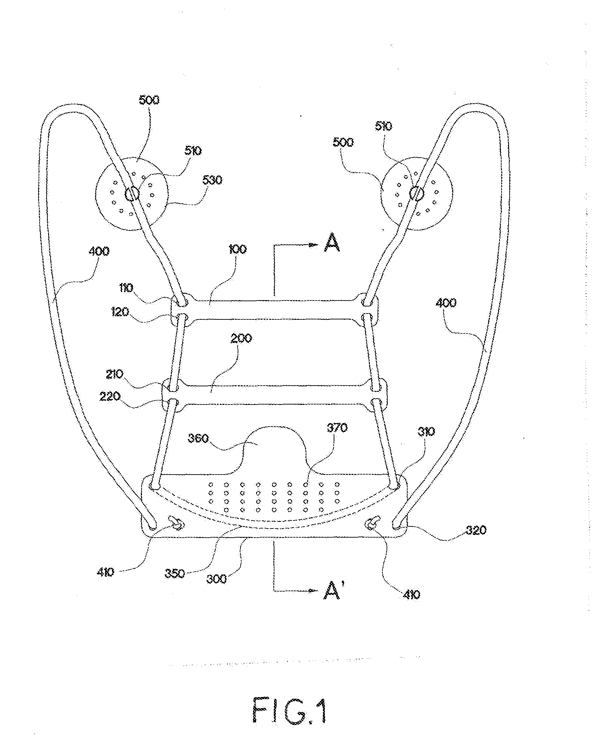

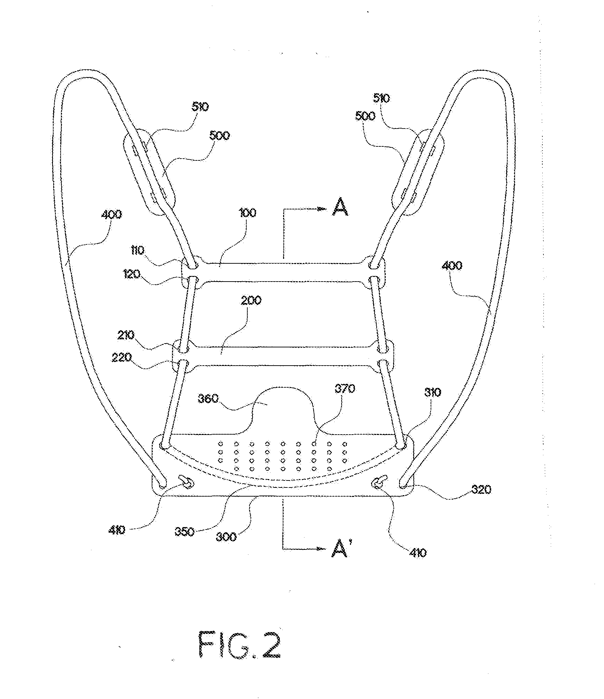

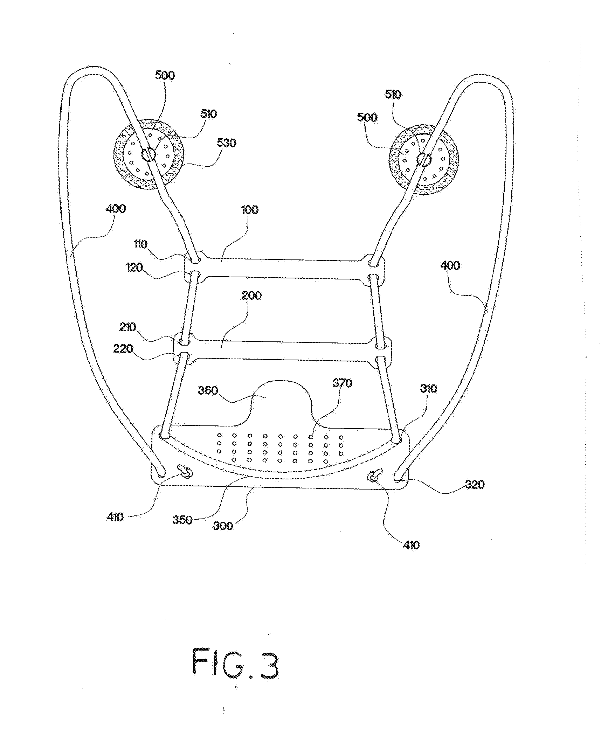

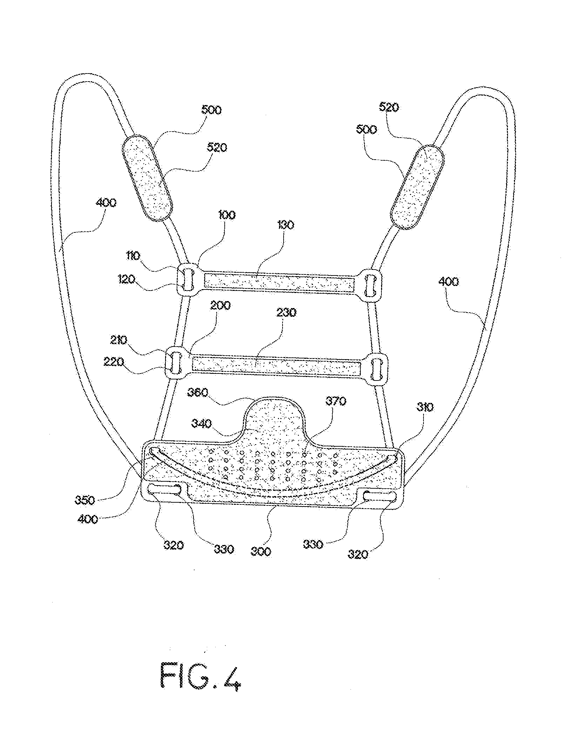

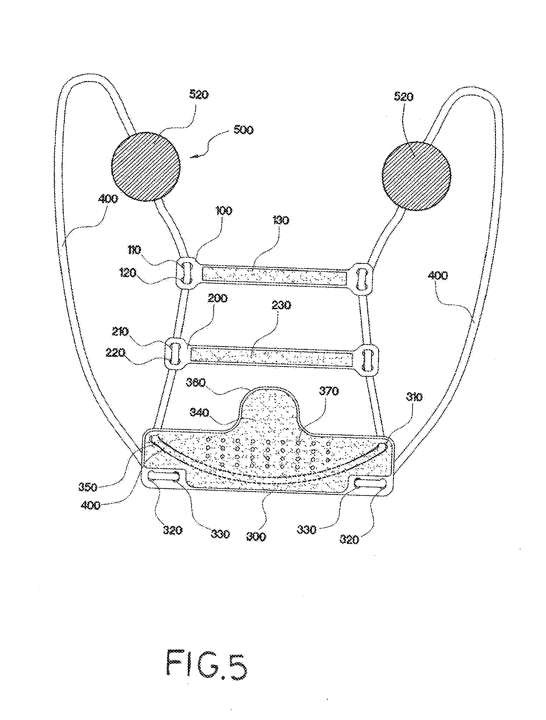

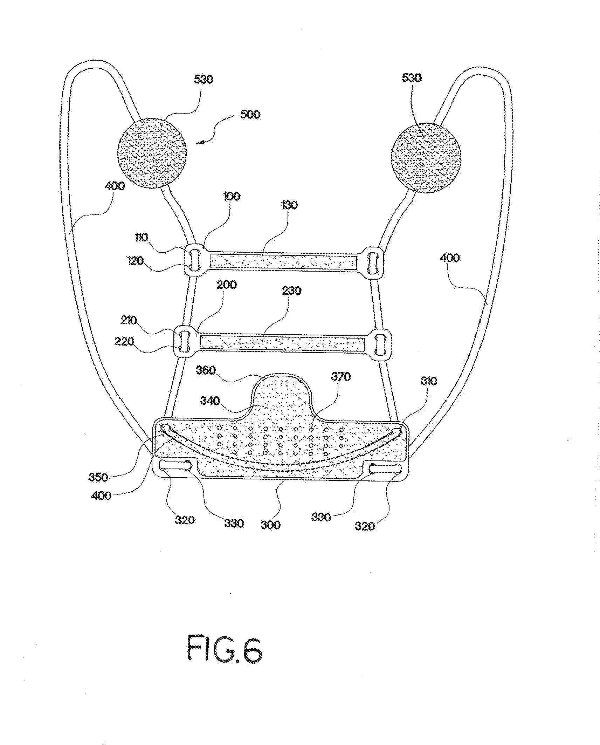

FIGS. 1 through 3 are front views illustrating a device for preventing mouth opening during sleep according to embodiments of the present invention. FIGS. 4 through 6 are rear views illustrating the device for preventing mouth opening during sleep according to embodiments of the present invention.

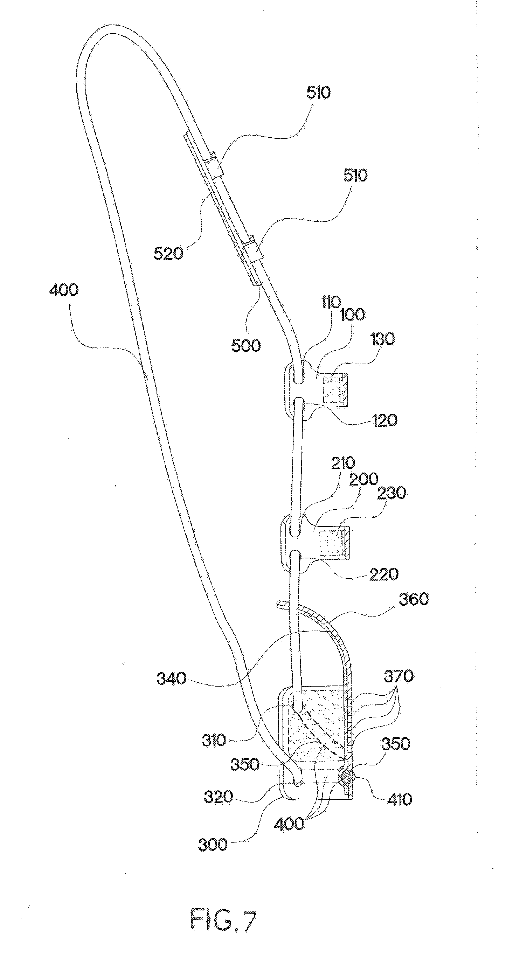

In addition, FIG. 7 is a side sectional view taken along line A-A' of FIG. 1 to illustrate the device for preventing mouth opening during sleep according to an embodiment of the present invention.

According to the embodiments, the device is provided for preventing mouth opening during sleep. The device includes: an upper lip supporter 100 which is disposed firmly above an upper lip and has a long, narrow, thin, and curved plate shape; a lower lip supporter 200 which is disposed firmly below a lower lip and has a long, narrow, thin, and curved plate shape; a lower jaw supporter 300 having a protruded fixing unit 360 which supports a lower jaw, is disposed firmly on a face, and has a center part protruding forward from the lower jaw and bent upward forming a gentle curve; and a coupling medium 400 which couples and fixes the supporters firmly on the face of a wearer.

First upper lip supporter coupling holes 110 are disposed in upper portions of both side ends of the upper lip supporter 100, and second upper lip supporter coupling holes 120 are disposed in lower portions of the both side ends of the upper lip supporter 100. The coupling medium 400 which is a strap formed of elastic span fabric may be sequentially inserted into the coupling holes to couple the upper lip supporter 100 with the coupling medium 400. In addition, a wearing sub-supporter 130 formed of thin woven cotton fabric is disposed on a rear surface of the upper lip supporter 100.

First lower lip supporter coupling holes 210 are disposed in upper portions of both side ends of the lower lip supporter 200, and second lower lip supporter coupling holes 220 are disposed in lower portions of the both side ends of the lower lip supporter 200. The strap-shaped coupling medium 400 formed of the elastic span fabric may be sequentially inserted into the coupling holes to couple the lower lip supporter 200 with the coupling medium 400. In addition, a wearing sub-supporter 230 formed of the thin woven cotton fabric is disposed on a rear surface of the lower lip supporter 200.

The lower jaw supporter 300 may be horizontally disposed in firm contact with a lower surface of the lower jaw of a wearer by bringing the protruded fixing unit 360 into firm contact with the lower jaw so that the protruded fixing unit 360 can be supported at a front side of the lower jaw. Lower jaw supporter coupling holes 310 are disposed at both upper side ends of the lower jaw supporter 300, and first length adjustment holes 320 and second length adjustment holes 330 are disposed at both lower side ends of the lower jaw supporter 300. The first length adjustment holes 320 are disposed in outer portions of the lower jaw supporter 300, and the second length adjustment holes 330 are disposed in inner portions of the lower jaw supporter 300. In addition, a guideline 350 which is a groove having a predetermined depth is disposed on a rear surface of the lower jaw supporter 300, the guideline 300 extending from one of the lower jaw supporter coupling holes 310, terming a semicircular are drooping downward, and extending toward the other of the lower jaw supporter coupling holes 310. Along the guideline 350, a center part of the coupling medium 400 is fixed to the rear surface of the lower jaw supporter 300 in a curvature convex toward a neck, and a wearing sub-supporter 340 is attached to the lower jaw supporter 300 so that the coupling medium 400 is fixed on the lower jaw supporter. Therefor, the center part of the coupling medium 400, inserted into the groove of the guideline 350 and fixed, not only prevents the lower jaw supporter 300 from being overturned or twisted because of a lever rule when the device for preventing mouth opening during sleep is worn, but also disposes the protruded fixing unit 360 firmly on the front side of the lower jaw. In addition, by attaching the wearing sub-supporter 340 formed of natural cotton fabric to the rear surface of the lower jaw supporter 300, the coupling medium 400 fixed to the groove of the guideline 350 may be covered entirely and a position of the coupling medium 400 may be fixed.

Therefore, the wearing sub-supporter 340 is disposed covering the guideline 350 disposed on the rear surface of the lower jaw supporter 300 and the coupling medium 400 disposed between the lower jaw supporter coupling holes 310. In this case, on the rear surface of the lower jaw supporter 300, the wearing sub-supporter 340 does not cover the length adjustment holes 320 and 330, since a length of the coupling medium 400 and a degree to which the device is disposed firmly on the face may be adjusted by loosening or tightening the coupling medium 400 through the length adjustment holes 320 and 330. In addition, the protruded fixing unit 360 protruding forward and bent upward forming a gentle curve is disposed in a center part of the lower jaw supporter 300, and a plurality of lower jaw supporter holes 370 are disposed in the lower jaw supporter 300 for a facial skin, an air permeability, and an improved appearance. Although FIGS. 1 through 6 illustrate the device in an unworn state, the lower jaw supporter 300 is fixed firmly and horizontally on the lower surface of the lower jaw when the device is worn, and thus the protruded fixing unit 360 is disposed on the front side of the lower jaw of the wearer. The lower jaw supporter 300 in entirety may have a shape of a curved surface protruding in the middle.

FIG. 7 is a side sectional view of the device in a state when each supporter of the device has a shape of a curved surface protruding in the middle.

Meanwhile, a strap may form the coupling medium 400 in entirety. First of all, when the strap is used as the coupling medium 400, the center part of the coupling medium 400, as described above, is fixed to the rear surface of the lower jaw supporter 300 in a circular arc shape. Both side ends of the coupling medium 400 are inserted into the lower jaw supporter coupling holes 310, passed from the rear surface to a front surface of the lower jaw supporter 300, and coupled with each other. The both side ends are sequentially and alternately inserted into the second lower lip supporter coupling holes 220, the first lower lip supporter coupling holes 210, the second upper lip supporter coupling holes 120, and the first upper lip supporter coupling holes 110. In this case, the coupling medium 400 may be passed alternately through front surfaces and the rear surfaces of the supporters so that the coupling medium 400 comes into contact with the facial skin of the wearer, because a direct contact between a synthetic resin and the facial skin may be avoided and the strap-shaped coupling medium 400 may come into firm contact with the facial skin when the synthetic resin is used for the supporters.

The both side ends inserted into the first upper lip supporter coupling holes 110 are left with an enough length so that the coupling medium 400 is hung on both earflaps of the wearer. Then, the both side ends are sequentially and alternately inserted into the first length adjustment holes 320 and the second length adjustment holes 330 disposed at the both lower side ends of the lower jaw supporter 300. The first length adjustment holes 320 are disposed in the outer portions of the lower jaw supporter 300, and the second length adjustment holes 330 are disposed in the inner portions of the lower jaw supporter 300. Then, the both side ends are inserted into the second length adjustment holes 330 toward the front surface. By forming the both side ends of the coupling medium 400 into stoppers 410 having a knot shape, the both side ends may not be separated from the second length adjustment holes 330. The device may be worn and disposed firmly on the face of the wearer when parts of the coupling medium 400 which are inserted into the second length adjustment holes 330, are pulled, or when the length of the coupling medium 400 is adjusted by loosening the strap disposed on front surfaces of the first length adjustment holes 320.

In addition, since the supporters are coupled with the single coupling medium 400 and form the single device in entirety, the supporters may be moved conveniently by being pushed and pulled upward and downward along the coupling medium 400, and distances between the supporters may be adjusted conveniently, regardless of a shape and a size of the face of the wearer and regardless of an age and a sex of the wearer.

Meanwhile, when the device is worn during sleep and the coupling medium 400 is disposed on a protruded cheekbone of the face, the coupling medium 400 has the possibility of leaving a mark on the facial skin by pressing the facial skin. Therefore, mark preventing units 500 may be inserted and disposed under the coupling medium 400 so that a mark is not left on the protruded cheekbone of the face even after the device is worn during sleep for a long period of time. In addition, mark preventing sub-supporters 520 formed of the natural cotton fabric may be disposed on inner surfaces of the mark preventing units 500 for comfortable wearing. In addition, pairs of coupling medium fixing protrusions or single coupling medium fixing protrusions 510 disposed on outer surfaces of the mark preventing units 500 may fix the coupling medium 400 firmly so that the coupling medium 400 is not separated from the coupling medium fixing protrusions 510 once the coupling medium 400 is inserted into grooves of the coupling medium fixing protrusions 510. In case of the pair of coupling medium fixing protrusions 510, a distance between the coupling medium fixing protrusions 510 may increase upward. In addition, if mark preventing protection covers 530 are disposed on outer sides of the single coupling medium fixing protrusions 510, the wearer may feel comfortable in the protruded cheekbone even when the device is worn for a long period of time.

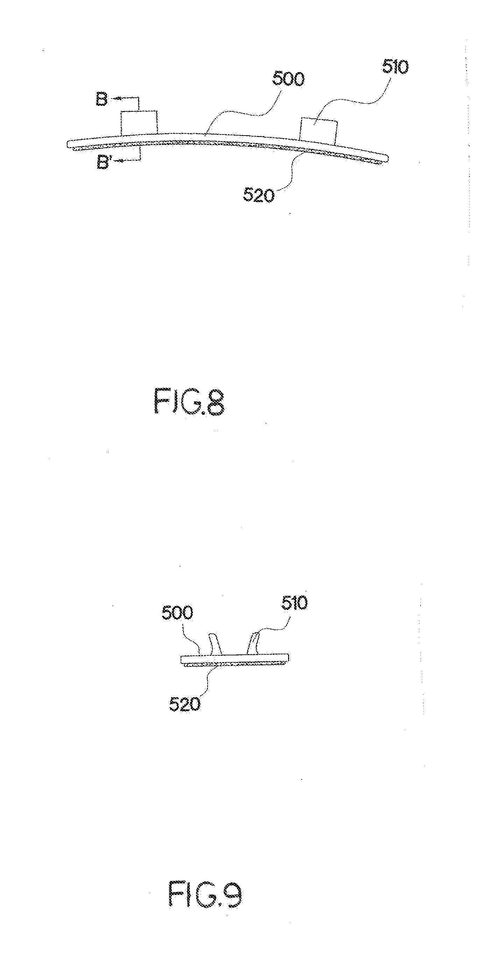

FIG. 8 is a cross sectional view illustrating mark preventing units of the device according to an embodiment of the present invention, and FIG. 9 is a vertical sectional view taken along line B-B' of FIG. 9.

The mark preventing sub-supporters 520 are attached to the inner surfaces of the mark preventing units 500 having a thin and curved plate shape to be fixed firmly on the protruded cheekbone. The coupling medium fixing protrusions 510 having a predetermined distance each other are disposed on one side or both sides of the outer surfaces.

In this case, upper portions of the coupling medium fixing protrusions 510 may be opened, and distances between the upper portions of the coupling medium fixing protrusions 510 may be larger than distances between lower portions of the coupling medium fixing protrusions 510, so that the coupling medium 400 is firmly inserted into the coupling medium fixing protrusions 510.

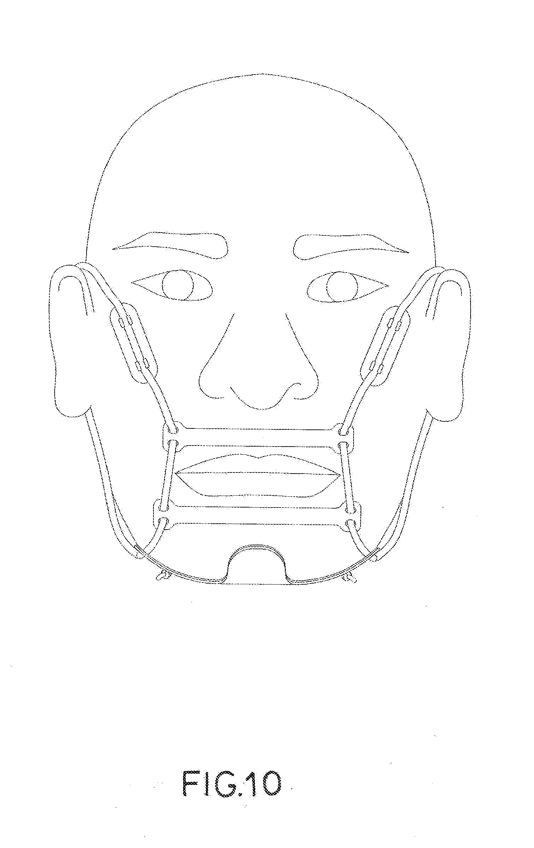

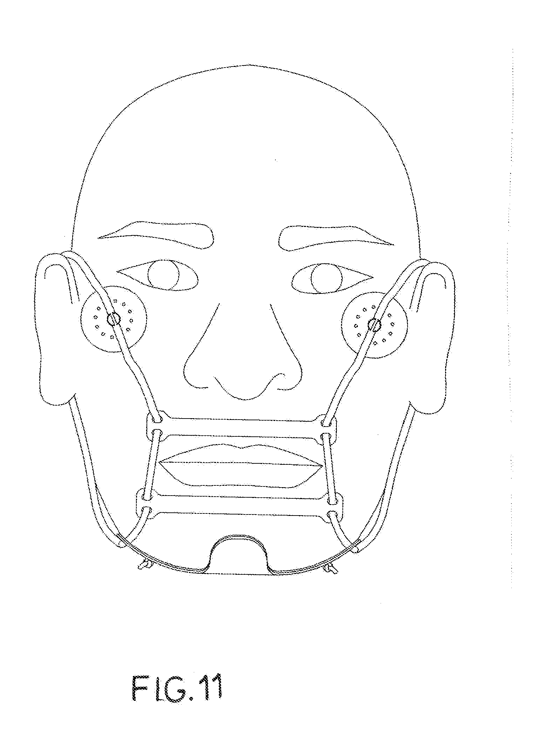

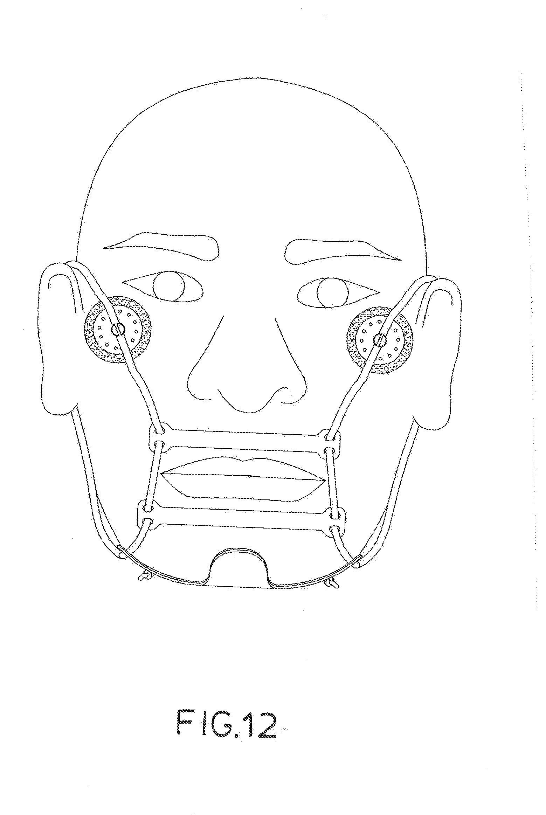

FIGS. 10 through 12 are views illustrating states of the device worn on the face according to the embodiments of the present invention.

The wearer, after wearing the device and hanging the coupling medium 400 on ears, disposes each supporter above the upper lip, below the lower lip, and on the lower surface of the lower jaw. Then, the wearer adjusts the length of the coupling medium 400 by pulling or untying the side ends inserted into the length adjustment holes 320 and 330 so that the device is disposed firmly on the facial skin. In this case, the mark preventing units 500 may be attached later on the cheekbone of the face.

When the device is worn during sleep, mouth breathing is inhibited, nose breathing is induced, such sleep disorders as xerostama, stertorous respiration, and sleep apnea are alleviated, and oral muscles are strengthened.

In addition, the device minimizes discomfort by minimizing an area of the facial skin which comes into contact with the device when the device is worn. Although the device efficiently prevents a mouth from opening during sleep, the device is manufactured inexpensively.

In addition, the device fits the face of the wearer and is easily worn and separated, since the supporters of the device are coupled into one by means of the coupling medium and distances between the supporters are easily adjusted.

While this invention has been particularly shown and described with reference to preferred embodiments thereof, it will be understood by those skilled in the art that various changes in form and details may be made therein without departing from the spirit and scope of the invention as defined by the appended claims, The preferred embodiments should be considered in descriptive sense only and not for purposes of limitation. Therefore, the scope of the invention is defined not by the detailed description of the invention but by the appended claims, and all differences within the scope will be construed as being included in the present invention.

## Claims

1. A device for preventing mouth opening during sleep, comprising:
an upper lip supporter comprising at least one coupling hole in each of upper and lower side portions, wherein the upper lip supporter is configured to be firmly disposed above an upper lip and has a thin plate shape;
a lower lip supporter comprising at least one coupling hole in each of upper and lower side portions, the lower lip supporter is configured to be firmly disposed below a lower lip and has a thin plate shape;
a lower jaw supporter having a thin plate shape and configured to be firmly disposed on a lower surface of a lower jaw, wherein the lower jaw supporter comprises a coupling hole in each upper side portion, at least one length adjustment hole in each lower side portion in a length direction of the lower side portion, and a protruded fixing unit in a center part, the protruded fixing unit being protruded toward a front side of the lower jaw and bent upward forming a gentle curve along the lower jaw so that the lower jaw supporter does not move toward a neck; and
a coupling medium, wherein a center part of the coupling medium is disposed on a rear surface of the lower jaw supporter to support the lower jaw supporter, wherein each side end of the coupling medium is inserted into the coupling hole of the lower jaw supporter toward a front side of the lower jaw supporter and then sequentially inserted into the coupling holes of the lower lip supporter and the upper lip supporter to connect the lower lip supporter and the upper lip supporter upward, and the each side end of the coupling medium is inserted into the length adjustment hole disposed in each lower portion of the lower jaw supporter so that a length of the coupling medium hung on each ear is adjusted and the coupling medium is disposed firmly on face.

2. The device of claim 1, wherein each coupling hole disposed on each supporter has a length larger than a width, wherein the length adjustment hole disposed on the lower jaw supporter has a width larger than a length.

3. The device of claim 2, wherein a stopper is disposed at the each side end of the coupling medium so that the each side end of the coupling medium inserted into the length adjustment hole is not separated from the length adjustment hole.

4. The device of claim 1, wherein a guideline is disposed on the rear surface of the lower jaw supporter to receive the center part of the coupling medium, wherein the guideline comprises a groove having a predetermined depth and a semicircular arc shape from the coupling hole toward the neck.

5. The device of claim 1, wherein a plurality of holes are disposed across the lower jaw supporter to allow flow of air and improve an appearance of the device.

6. The device of claim 1, wherein the each supporter has a forwardly-curved center part so as to be firmly disposed on the face.

7. The device of claim 1, wherein the each supporter is formed of an elastic and soft synthetic resin material, and the coupling medium is a strap formed of a flexible fabric and having a predetermined thickness.

8. The device of any one of claims 1 to 7, wherein a wearing sub-supporter formed of a natural cotton fabric or a soft velvet woven fabric is disposed on a rear surface of each supporter to reduce an irritation from a dermal contact.

9. The device of claim 8, further comprising a mark preventing unit having a polygonal or oval thin-plate shape,
wherein an inner surface of the mark preventing unit comes into contact, with a skin of a cheekbone,
a center part of the mark preventing unit is convexly protruded correspondingly to a protruded shape of the cheekbone,
a pair of coupling medium fixing protrusions are disposed on both sides of the upper portion of the mark preventing unit, or a single coupling medium fixing protrusion is disposed on a side of the upper portion of the mark preventing unit, for fitting the coupling medium therein.

10. The device of claim 9, wherein the pair of coupling medium fixing protrusions are outwardly inclined so that a distance between the pair of coupling medium fixing protrusions increases upward.

11. The device of claim 9, wherein the single coupling medium fixing protrusion comprises a mark preventing protection cover in an outer portion for comfortable wearing.

12. The device of claim 10 or 11, further comprising a mark preventing sub-supporter formed of the natural cotton fabric or the soft velvet woven fabric and disposed on the bottom surface of the mark preventing unit.
